# EUROPÄISCHE PATENTANMELDUNG

(11) **EP 1 518 548 A2**
(43) Veröffentlichungstag der Anmeldung: **30.03.2005**
(21) Anmeldenummer: 04021689.7
(22) Anmeldetag: 13.09.2004
(51) Int. Cl.: A61K 7/42, A61K 7/48

(54) **Lichtschutzzubereitung mit einem Gehalt an reflektierenden Pigmenten (Mikrospiegeln)**

(30) Priorität: 26.09.2003 DE 10344889
(71) Anmelder: Beiersdorf AG, 20253 Hamburg (DE)
(72) Erfinder: Eitrich, Anja, 22587 Hamburg (DE); Wepf, Roger, Dr., 22455 Hamburg (DE); Dippe, Rixa, Dr., 20255 Hamburg (DE)

(57) **Zusammenfassung**

Kosmetische oder dermatologische Zubereitung mit einem Gehalt an reflektierenden Festkörpersubstanzen, welche in die drei Raumrichtungen A, B und C die Ausdehnungen a, b und c aufweisen, wobei
- a ≤ 1 µm,
- 10 µm ≤ b ≤ 70 µm und
- 10 µm ≤ c ≤ 70 µm
gewählt werden.

## Beschreibung

Die vorliegende Erfindung betrifft eine kosmetische oder dermatologische (Lichtschutz-) Zubereitung mit einem Gehalt an bestimmten reflektierenden Pigmenten ("Mikrospiegeln").

Neben den positiven A uswirkungen d es S onnenlichtes, w ie d em allgemeinen W ohlbefinden, der Bildung von Vitamin D3 und der Aknebehandlung, gibt es auch negative Auswirkungen, denen es entgegenzuwirken gilt.

Setzt man die Haut zu lange der Sonne oder einer künstlichen Strahlenquelle aus, so entwickelt sich nach einer Latenzzeit von 2 bis 3 Stunden eine gegen die unbestrahlte Haut stark abgegrenzte Hautrötung, das Erythema solare. Bei dem so entstehenden Sonnenbrand unterscheidet man zwischen
■ 1. Grad: Erythem (Rötung, Wärmegefühl)
   klingt nach 2 bis 3 Tagen wieder ab und verschwindet unter gleichzeitig zunehmender Pigmentierung,
■ 2. Grad: Blasenbildung
   auf der Haut bilden sich Blasen mit Brennen und Jucken, die Oberhaut wird flächig abgestoßen
■ 3. Grad: Zellschädigung
   es treten tiefgehende Zellschädigungen auf, der Körper reagiert mit Fieber, die Oberhaut wird großflächig abgestoßen.

Der 2. und 3. Grad werden auch als Dermatitis solare bezeichnet.

Die Bildung des Erythems ist abhängig von der Wellenlänge. Der Erythembereich des UV-B liegt zwischen 280 nm und 320 nm.

Etwa 90 % der auf die Erde gelangenden ultravioletten Strahlung besteht aus UV-A-Strahlen mit einer Wellenlänge zwischen 320 nm und 400 nm. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Man hat lange Zeit fälschlicherweise angenommen, daß die langwellige UV-A-Strahlung nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

Der Sonnenbrand bzw. das Lichterythem sind die akuten Erscheinungsformen der Lichteinwirkung. Darüber hinaus zeigt sich aber an besonders stark exponierten Hautpartien (Gesicht, Nacken, Hände) mit zunehmendem Alter aufgrund der damit in Zusammenhang stehenden hohen Gesamtdosis eine durch Strahlung hervorgerufene (aktinische) Veränderung der Haut. Die auffälligste chronische Lichtschädigung der Haut ist die aktinische oder senile Elastose. Makroskopisch äußert sie sich in einer Verdickung und Vergröberung der Haut, Faltenbildung, Verlust der Elastizität, Auftreten von gelblich druchschimmerden Einlagerungen und unregelmäßigen Pigmentanhäufungen. Die Oberhaut wird stellenweise dünn und zeigt warzige Wucherungen, die Lederhaut verliert ihre Elastizität und Spannung, das Wasserbindungsvermögen wird verringert. Zu den chronischen Lichtschäden, die als Spätfolgen auftreten, gehören ferner das maligne Melanom und im letzten Stadium die aktinische Keratose.

Da die Beiträge der verschiedenen Wellenlängenbereiche des UV-Lichtes noch nicht vollständig geklärt sind, ist vorbeugender Schutz sowohl für den UV-A als auch den UV-B-Bereich, beispielsweise durch Auftrag von Lichtschutzfiltersubstanzen in Form einer kosmetischen oder dermatologischen Formulierung auf die Haut, von entscheidender Bedeutung. Kosmetische Zubereitungen sollten die kritischen UV-A-Strahlen grundsätzlich stark absorbieren, nicht nur zum Schutz empfindlicher Haut beim Sonnenbaden, sondern auch für den allgemeinen Schutz bis hin zur Anwendung in einer normalen Hautcreme, da Hautalterung und Risiko des Hautkrebses wesentlich von diesem Teil des UV-Lichtes beeinflußt werden.

Der Nutzen eines Sonnenschutzpräparates besteht darin, die Zeit zu verlängern, die ein Verbraucher in der Sonne verbleiben kann, ohne Lichtschäden davonzutragen. Dabei sollte die Filterwirkung insbesondere für den UV-B-Bereich der individuellen Empfindlichkeit der Verbrauchers und der Intensität der Sonnenbestrahlung angepaßt sein.

Die Schwächung der Intensität des eingestrahlten Lichts und damit die Wirksamkeit einer Sonnenschutzformulierung sind unter anderem abhängig von der Schichtdicke der aufgetragenen Filtersubstanz. Mit steigender Schichtdicke erhöht sich auch die Wirksamkeit eines Sonnenschutzmittels. Besonders bei dünnflüssigen Zubereitungen wie Ölen oder alkoholischen Lösungen ist es deshalb notwendig, ab einer gewissen Grenzkonzentration an Filtersubstanz auch die Viskosität der Formulierung und damit die erreichbare Schichtdicke auf der Haut zu erhöhen. Es ist darüber hinaus nur begrenzt möglich, durch Erhöhung der Konzentration der UV-Filtersubstanz den Lichtschutzfaktor zu erhöhen.

Die mit einer speziellen Grundlage erreichte Schichtdicke ist ein sehr wichtiges Kriterium im Lichtschutz. Im allgemeinen nimmt der Lichtschutzfaktor in der Reihenfolge Öl < alkoholische Lösung < flüssige Emulsion < Creme < Paste zu. Ein weiterer wichtiger Parameter in diesem Zusammenhang ist ferner die Haftfähigkeit bzw. Wasserfestigkeit der Formulierungen a uf der Haut. Die meisten Sonnenschutzmittel werden in Wassernähe oder bei sportlicher Betätigung (Schwitzen) angewendet. Ein wasserfestes Sonnenschutzmittel kann den Anwender nicht nur während des Badens, sondern auch nach dem Baden vor Sonnenbrand schützen, sofern es nicht zu stark abgewaschen wird.

Der Stand der Technik kennt selbstverständlich eine Vielzahl verschiedener Lichtschutzformulierungen mit den unterschiedlichsten Lichtschutzfaktoren und Anwendungseigenschaften. Nachteil dieser üblichen Sonnenschutzmittel ist, daß der Anwender nach dem Eincremen weder erkennen kann, ob er alle der Strahlung ausgesetzten Körperteile mit einer schützenden Schicht bedeckt hat noch ob die Abdeckung der Haut in ausreichendem Maß erfolgt ist, d. h. ob die Schichtdicke ausreichend gewählt wurde. Ein weiterer Nachteil ist, daß der Anwender nicht verfolgen kann, wie sich die Schichtdicke des Sonnenschutzmittels verändert, beispielsweise durch Kontakt mit Wasser oder mechanische Belastungen wie Abtrocknen etc., und wann dementsprechend ein neues Eincremen erforderlich geworden ist.

Dies kann im schlimmsten Fall zu einer verringerten Schutzwirkung der Produkte führen.

Insbesondere für Kinder- und Babyprodukte ist e ine h ohe S chutzleistung d er Z ubereitungen von besonderer Bedeutung, da Kinder aufgrund der geringeren Lichtschwiele (Verdickung der Hornschicht) und des verminderten Eigenschutzes besonders anfällig für Sonnenbrände sind und zudem oft stundenlang am oder im Wasser spielen. Ferner ist das Hautkrebsrisiko eines Erwachsenen umso höher, je mehr Sonnenbrände er als Kind erlitten hat.

Gleichzeitig ist es aber häufig problematisch, Kinder ausreichend einzucremen, da diese meist sehr unruhig oder ungeduldig sind und Lichtschutzzubereitungen mit hohem Lichtschutzfaktor sich zudem wegen ihrer in der Regel höheren Viskosität nur schlecht verteilen lassen. Darüberhinaus ist es für die Eltern im allgemeinen nur schwer zu erkennen, wann das Sonnenschutzmittel abgerieben oder abgewaschen ist und die Kinder erneut eingecremt werden müßten.

Aufgabe der vorliegenden Erfindung war dementsprechend, den Nachteilen des Standes der Technik abzuhelfen und Möglichkeiten zu finden, kosmetische und dermatologische Sonnenschutzformulierungen auf der Haut zu visualisieren und so die Auftragung eines gleichmäßigen Films auf die Haut zu erleichtern.

Zwar beschreibt die DE-100 34 332-A1 die Verwendung von Effektpigmenten mit einer mittleren Partikelgröße von 15 µm bis 200 µm zur Sichtbarmachung von kosmetischen und dermatologischen Formulierungen auf der Haut. Ferner beschreibt die DE-100 35 512-A1 die Verwendung von Farbstoffen zur Sichtbarmachung von kosmetischen und dermatologischen Lichtschutzformulierungen auf der Haut.

Sowohl d ie E P-941 051-B1 als a uch die EP 1 136 059-A2 beschreiben darüber hinaus farbige Sonnenschutzemulsioen, die sich dadurch auszeichnen, daß die Farbe im wesentlichen verblaßt, wenn die Sonnenschutzemulsion trocknet, nachdem sie auf der Haut verteilt und/oder in die Haut eingerieben wurde. Allerdings bleibt auch hier die erzielte Wirkung hinter der erhofften zurück.

Des weiteren kennt der Stand der Technik Sonnenschutzmittel mit Ultraspektralschutz, welche einen Komplettschutz gegen das gesamte, die Erde erreichende Sonnenspektrum bieten sollen (EP-898 955-A2).

Und selbstverständlich ist die Verwendung von sog. "physikalischen Filtersubstanzen" (Pigmenten), welche je nach Größe und Form bestimme Teile des eingestrahlten Sonnenlichtes reflektieren und so eine Filterwirkung herbeiführen, seit geraumer Zeit an sich bekannt.

Allerdings konnte der Stand der Technik nicht den Weg zur vorliegenden Erfindung weisen.

Es war nach all dem überraschend und für den Fachmann in keiner Weise vorhersehbar, daß
kosmetische oder dermatologische Zubereitungen mit einem Gehalt an reflektierenden Festkörpersubstanzen, welche in die drei Raumrichtungen A, B und C die Ausdehnungen a, b und c aufweisen, wobei
2. a ≤ 1 µm,
3. 10 µm ≤ b ≤ 70 µm und
4. 10 µm ≤ c ≤ 70 µm
gewählt werden,
den Nachteilen des Standes der Technik abhelfen würden.

Die erfindungsgemäßen Festkörpersubstanzen reflektieren sichtbares Licht und fungieren dementsprechend als "Mikrospiegel" bzw. "Mikrospiegelfolien". Sie sind kristallin und zeigen üblicherweise eine atomare Bruchfläche, an der sich das eingestrahlte Licht spiegelt. Vorteilhaft im Sinne der vorliegenden Erfindung sind insbesondere Festkörpersubstanzen, welche keinerlei Absorption zeigen, welche sich dementsprechend unter Bestrahlung nicht erwärmen.

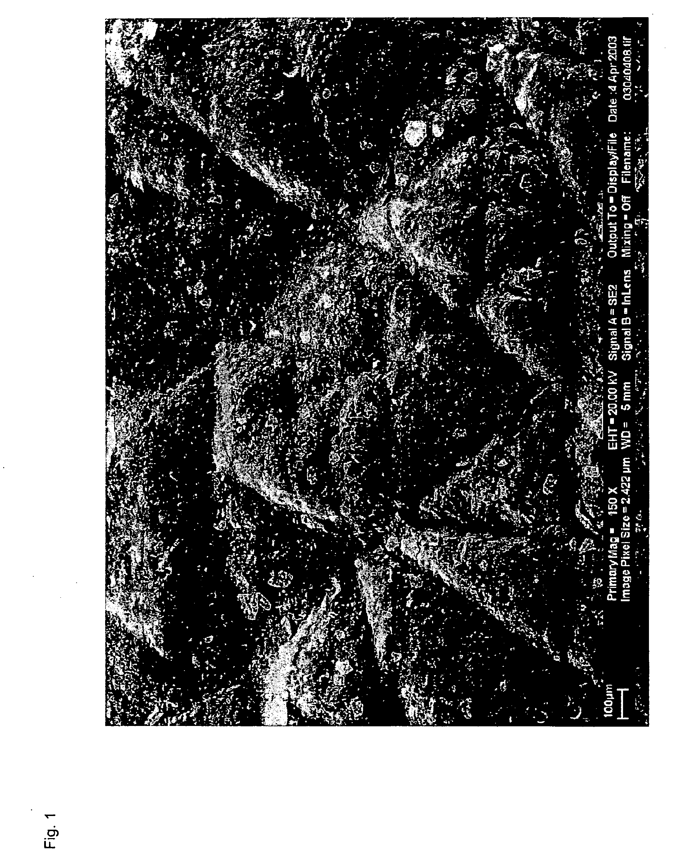

Es war insbesondere erstaunlich, daß die erfindungsgemäßen Festkörpersubstanzen - ebenso wie die kosmetische Zubereitung selbst - beim Verstreichen des Produktes auf der Haut homogen auf dieser verteilt werden. Ein besonderer Vorteil der erfindungsgemäßen Festkörpersubstanzen ist, daß sich diese nicht in den (feinen) Falten der Haut sammeln, sondern vielmehr auf den zwischen den Hautfalten liegenden Hautarealen gleichmäßig verteilt werden. Dabei bilden sich ferner überraschend keine Festkörperaggregate aus, sondern die Festkörperplättchen liegen nach dem Verstreichen des Produktes einzeln separiert auf der Haut (siehe Fig. 1).

Die erfindungsgemäßen Zubereitungen selbst zeigen üblicherweise keine oder nur geringe spiegelnde Eigenschaften, da eine gleichgerichtete Anordnung der Mikrospiegel entscheidend für das (makrokopische) Reflektionsverhalten der Zubereitung ist. In den erfindungsgemäßen Zubereitungen liegen die Mikrospiegelfolien aber unregelmäßig ausgerichtet und darüber hinaus möglicherweise auch eher leicht aggregiert vor. Ferner wirken die erfindungsgemäßen Festkörpersubstanzen auch dann nicht refektierend, wenn sie von der Zubereitung bedeckt sind. Beim Verteilen des Produktes auf der Haut werden die erfindungsgemäßen Festkörpersubstanzen durch Physisorption bzw. Adhäsion festgehalten, so daß sie glatt ausgestrichen und flach ausgerichtet und ferner von der kosmtischen Zubereitung befreit werden können.

Erst diese Eigenschaft der erfindungsgemäßen Festkörpersubstanzen ermöglicht deren Spiegelwirkung auf der Hautoberfläche.

Die erfindungsgemäßen Substanzen können sowohl einzeln als auch im Gemisch vorliegen. Die Gesamtmenge der erfindungsgemäßen Substanzen wird vorteilhaft aus dem Bereich von 0,01 Gew.-% bis 20 Gew.-%, vorzugsweise von 0,05 bis 15 Gew.-%, insbesondere von 0,05 bis 5 Gew.-% gewählt, jeweils bezogen auf das Gesamtgewicht der Zubereitungen.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch Mikroemulsionen, Stifte, Schäume (sog. Mousse), Feststoff-Emulsionen (d. h. Emulsionen, welche durch Feststoffe stabilisiert sind, z. B. Pickering-Emulsionen), sprühbare Emulsionen oder Hydrodispersionen sein. Des weiteren können die Zubereitungen vorteilhaft auch ölfreie und/oder wäßrige/alkoholische Lösungen sein. Es ist erfindungsgemäß bevorzugt, wenn die Formulierungen wasserfest sind.

Auch (makroskopisch) zwei- oder mehrphasige Systeme sind erfindungsgemäß vorteilhaft. "Zwei- oder mehrphasig" im Sinne der vorliegenden Erfindung bedeutet, daß zwei oder mehr Phasen separat übereinander geschichtet vorliegen. Es ist besonders vorteilhaft im Sinne der vorliegenden Erfindung, wenn mindestens eine der makroskopisch sichtbaren Phasen eine (W/O-, O/W-, Mikro-) Emulsion darstellt. Die Emulsion wird bei dieser (makroskopischen) Betrachtung als eine Phase wahrgenommen, obwohl es dem Fachmann natürlich bekannt ist, daß Emulsionen an sich aus zwei oder mehr miteinander homogenisierten Phasen gebildet werden. Die "Emulsionsphase" ist langzeitstabil, so daß es auch über einen längeren Zeitraum (Monate, Jahre) nicht zu einer Entmischung beziehungsweise Phasenauftrennung innerhalb der Emulsion kommt.
Die makroskopisch sichtbaren Phasen bzw. Schichten lassen sich vorteilhaft - zum Beispiel durch Schütteln - kurzfristig zu einer homogenen Emulsion emulgieren, welche aber nicht langzeitstabil ist, sondern sich vielmehr über einen Zeitraum von Minuten, Stunden oder Tagen wieder zu zwei oder mehr übereinander geschichteten Phasen entmischt. Es ist besonders vorteilhaft im Sinne der vorliegenden Erfindung, wenn mindestens eine der makroskopisch sichtbaren Phasen eine Mikroemulsion und mindestens eine andere der makroskopisch sichtbaren Phasen eine Ölphase darstellt.

Es ist erfindungsgemäß besonders bevorzugt, wenn die kosmetischen Zubereitungen Inhaltsstoffe enthalten, die eine gute Verteilbarkeit des Produktes auf der Haut gewährleisten.

### Sprühbare Emulsionen, insbesondere Mikroemulsionen

Besonders vorteilhaft im Sinne der vorliegenden Erfindung sind sprühbare O/W-Emulsionen, insbesondere O/W-Mikroemulsionen.

Die T röpfchendurchmesser d er g ewöhnlichen "einfachen", a Iso n ichtmultiplen E mulsionen liegen im Bereich von ca 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 0,5 µm bis ca. 1 µm liegen, sind, wiederum ohne färbende Zusätze, bläulichweißgefärbt und opak. Solche "Makroemulsionen" haben für gewöhnlich eine hohe Viskosität.

Der Tröpfchendurchmesser von Mikroemulsionen im Sinne der vorliegenden Erfindung dagegen liegt im Bereich von etwa 50 bis etwa 500 nm. Derartige Mikroemulsionen sind bläulichweiß gefärbt bis transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von "Makroemulsionen". Ein weiterer Vorteil ist, daß sie aufgrund ihrer niedrigen Viskosität besser versprühbar sind. Werden Mikroemulsionen als Kosmetika verwendet, zeichnen sich entsprechende Produkte durch hohe kosmetische Eleganz aus.

Erfindungsgemäß vorteilhaft sind insbesondere O/W-Mikroemulsionen, welche mit Hilfe der sogenannten Phaseninversionstemperatur-Technologie erhältlich sind und mindestens e inen Emulgator (Emulgator A) enthalten, welcher gewählt wird aus der Gruppe der Emulgatoren mit folgenden Eigenschaften:
- ihre Lipophilie ist abhängig von der Temperatur, dergestalt daß durch Erhöhung der Temperatur die Lipophilie zunimmt und durch Senkung der Temperatur die Lipophilie des Emulgators abnimmt.
Vorteilhafte Emulgatoren A sind z. B. polyethoxilierte Fettsäuren (PEG-100 Stearat, PEG-20 Stearat, PEG-150 Laurath, PEG-8 Distearat und dergleichen) und/oder polyethoxilierte Fettalkohole (Cetearath-12, Cetearath-20, Isoceteth-20, Beheneth-20, Laureth-9 etc.) und/oder Alkylpolyglycoside (Cetearyl Glycoside, Stearyl Glycoside, Palmityl Glycoside etc.).

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Die Tröpfchengrößen liegen in der Regel zwischen 20 und 500 nm.

Es ist im Sinn der vorliegenden Erfindung gegebenenfalls vorteilhaft, weitere, nicht unter die Definition des Emulgators A fallende, W/O und/oder O/W-Emulgatoren zu verwenden, beispielsweise um die Wasserfestigkeit der Zubereitungen gemäß der vorliegenden Erfindung zu erhöhen. Hier können z. B. Alkylmethiconcopolyole und/oder Alkyl-Dimethiconcopolyole (insbesondere Cetyl Dimethicone Copolyol, Lauryl Methicone Copolyol), W/O-Emulgatoren (wie z. B. Sorbitanstearat, Glycerylstearat, Glycerolstearat, Sorbitanoleat, Lecithin, Glycerylisostearat, Polyglyceryl-3-Oleat, Polyglyceryl-3 Diisostearat, PEG-7-hydriertes Ricinusöl, Polyglyceryl-4-isostearat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Glycol Distearat, Polyglyceryl-3-dipolyhydroxystearat) und/oder Fettsäureester der Schwefel- oder P hosphorsäure ( Cetylphosphat, Trilaureth-4 Phosphat, Trioleth-8-Phosphat, Stearylphosphat, Cetearylsulfat etc.) verwendet werden.

Weitere vorteilhafte sprühbare O/W-Emulsionen im Sinne der vorliegenden Erfindung sind dünnflüssige kosmetische oder dermatologische Hydrodispersionen, welche mindestens eine Ölphase und mindestens eine Wasserphase enthalten, wobei die Zubereitung durch mindestens einen Gelbildner stabilisiert wird und nicht notwendigerweise Emulgatoren enthalten muß, aber einen oder mehrere Emulgatoren enthalten kann.

Vorteilhafte Gelbildner für derartige Zubereitungen sind beispielsweise Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 907, 910, 934, 940, 941, 951, 954, 980, 981, 1342, 1382, 2984 und 5984. ebenso die ETD-Typen 2020, 2050 und Carbopol Ultrez 10. Weitere vorteilhafte Gelbildner für derartige Zubereitungen sind Xanthan Gummi, Cellulose Derivate und Johannisbrotkernmehl.

Als mögliche (fakultative) Emulgatoren können ethoxilierte Fettalkohole oder ethoxilierte Fettsäuren (insbesondere PEG-100 Stearat, Ceteareth-20) und/oder andere nichtionische oberflächenaktive Substanzen verwendet werden.

Ferner vorteilhaft können die sehr dünnflüssigen bis sprühbaren Emulsionen auch W/O- bzw. Wasser-in-Silikonöl- (W/S-) Emulsionen sein. Insbesondere vorteilhaft sind W/O- bzw. W/S-Emulsionen, die
- mindestens einen Silikonemulgator (W/S) mit einem HLB-Wert ≤ 8 und/oder mindestens einen W/O-Emulgator mit einem HLB-Wert < 7 und
- mindestens einen O/W-Emulgator mit einem HLB-Wert > 10 enthalten.
Derartige Zubereitungen enthalten ferner mindestens 20 Gew.-% Lipide, wobei die Lipidphase vorteilhaft auch Silikonöle enthalten bzw. sogar ganz aus solchen bestehen kann.

Der oder die Silikonemulgatoren kann vorteilhaft aus der Gruppe der Alkylmethiconcopolyole und/oder Alkyl-Dimethiconcopolyole gewählt werden (z. B. Dimethiconcopolyole, welche von der Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden, Cetyl Dimethiconcopolyol [Goldschmidt AG / ABIL@ EM 90], Cyclomethicon Dimethiconcopolyol [Goldschmidt AG / ABIL® EM 97], Laurylmethiconcopolyol [Dow Corning Ltd. / Dow Corning® 5200 Formulation Aid], Octyl Dimethicon Ethoxy Glucosid [Firma Wacker].

Der oder die W/O-Emulgatoren mit einem HLB-Wert < 7 kann vorteilhaft aus folgender Gruppe gewählt werden: Sorbitanstearat, Sorbitanoleat, Lecithin, Glyceryllanolat, Lanolin, hydriertem Ricinusöl, Glycerylisostearat, Polyglyceryl-3-Oleat, Pentaerythrithylisostearat, Methylglucosedioleat, Methylglucosedioleat im Gemisch mit Hydroxystearat und Bienenwachs, PEG-7-hydriertes Ricinusöl, Polyglyceryl-4-isostearat, Hexyllaurat, Acrylat/ C₁₀₋₃₀-Alkylacrylat-Crosspolymer, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat.

Der oder die O/W-Emulgatoren mit einem HLB-Wert > 10 kann vorteilhaft aus folgender Gruppe gewählt werden: Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, L aureth-4 P hosphat, S tearinsäure, Propylenglycolstearat SE, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat im Gemisch mit PEG-100 Stearat, Ceteareth-3, Isostearylglycerylether, Cetylstearylalkohol im Gemisch mit Natrium Cetylstearylsulfat, PEG-40-Stearat, Glycol Distearat, Polyglyceryl-2-PEG-4-Stearat, Ceteareth-12, Ceteareth-20, Ceteareth-30, Methyl-glucosesesquistearat, Steareth-10, PEG-20-Stearat, Steareth-21, Steareth-20, Isosteareth-20, PEG-45/ Dodecylglycol-Copolymer, Glycerylstearat SE, Ceteth-20, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquiisostearat, Ceteth-10, Isoceteth-20, Glycerylstearat im Gemisch mit Ceteareth-20, Ceteareth-12, Cetylstearylalkohol und Cetylpalmitat, PEG-30-Stearat, PEG-40-Stearat, PEG-100-Stearat.

Ferner vorteilhaft sind wäßrig-alkoholische Lösungen. Sie können von 0 Gew.-% bis 90 Gew.-% Ethanol enthalten. Wäßrig-alkoholische Lösungen können im Sinne der vorliegenden Erfindung vorteilhaft auch Lösungsvermittler enthalten, wie z. B. PEG-40 oder PEG-60 hydrogeniertes Ricinusöl.

Die Zubereitungen gemäß der vorliegenden Erfindung können vorteilhaft auch als kosmetische oder dermatologische Tränkungslösungen, mit welchen insbesondere wasserunlösliche Substrate - wie z. B. gewebte oder nicht-gewebte Tücher - befeuchtet sind, verwendet werden. Derartige Tränkungslösungen sind vorzugsweise dünnflüssig, insbesondere sprühbar (wie z. B. PIT-Emulsionen, Hydrodispersionen, W/O-Emulsionen, Öle, wäßrige Lösungen etc.) und haben vorzugsweise eine Viskosität von weniger als 2000 mPa·s, insbesondere weniger als 1.500 mPa·s (Meßgerät: Haake Viskotester VT-02 bei 25 °C). Mit ihrer Hilfe sind beispielsweise kosmetische Sonnenschutztücher, Pflegetücher und dergleichen erhältlich, welche die Kombination eines weichen, wasserunlöslichen Materials mit der dünnflüssigen kosmetischen und dermatologischen Tränkungslösung darstellen.

### Öle

Die Zubereitungen gemäß der vorliegenden Erfindung können vorteilhaft auch als wasserfreie Öle oder Ölgele oder Pasten vorliegen. Vorteilhafte Öle sind z. B. synthetische, halbsynthetische oder natürliche Öle wie beispielsweise Rapsöl, Reisöl, Avocadoöl, Olivenöl, Mineralöl, Cocoglyceride, Butylene Gylcol Dicaprylat/Dicaprat, C₁₂₋₁₅ Alkylbenzoat, Dicaprylyl Carbonat, Octyldodekanol und dergleichen mehr. Als Ölgelbildner können verschiedenste Wachse mit einem Schmelzpunkt > 25°C verwendet werden. Ferner vorteilhaft sind Gelbildner aus der Gruppe der Aerosile, der Alkylgalaktomannane (z. B. N-Hance AG 200 und N-Hance AG 50 von der Fa. Hercules) und Polyethylene-Derivate.

### Schäume

Besonders vorteilhaft im Sinne der vorliegenden Erfindung sind ferner selbstschäumende, schaumförmige, nachschäumende oder schäumbare kosmetische und dermatologische Zubereitungen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise - durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische oder dermatologische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Deratige Zubereitungen enthalten im Sinne der vorliegenden Erfindung vorteilhaft ein Emulgatorsystem, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie M ono- u nd/oder T riethanolamin) neutralisiert s ind. B esonders vorteilhaft s ind b eispielsweise S tearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs e rhalten w ird). B esonders b evorzugt s ind C etyl- u nd C etylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

### Pickering- / feststoffstabilisierte Emulsionen

Besonders vorteilhaft im Sinne der vorliegenden Erfindung sind ferner kosmetische oder dermatologische Zubereitungen, welche nur durch feinstverteilte Feststoffteilchen stabilisiert sind. Solche "emulgatorfreien" Emulsionen werden auch als Pickering-Emulsionen bezeichnet.

In Pickering-Emulsionen kommt es zu einer Anreicherung des festen Stoffes an der Phasengrenze Öl/Wasser in Form einer Schicht, wodurch ein Zusammenfließen der dispersen Phasen verhindert wird. Von wesentlicher Bedeutung sind dabei insbesondere die Oberflächeneigenschaften der Feststoffpartikel, welche sowohl hydrophile als auch lipophile Eigenschaften zeigen sollten.

Vorteilhaft können die stabilisierenden Feststoffteilchen auch oberflächlich wasserabweisend behandelt ("gecoatet") sein, w obei e in a mphiphiler C harakter dieser F eststoffteilchen gebildet werden bzw. erhalten bleiben soll. Die Oberflächenbehandlung kann darin bestehen, daß die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffteilchen wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) bzw. Modifikation die verwendeten Feststoffteilchen vorliegen.

Vorzugsweise werden die mikrofeinen Feststoffteilchen aus der Gruppe der amphiphilen Metalloxidpigmente gewählt. Vorteilhaft sind insbesondere:
- Titandioxide (gecoatet und ungecoatet): z. B. Eusolex T-2000 von der Fa. Merck, Titandioxid MT-100 Z von der Fa. Tayca Corporation
- Zinkoxide z. B. Z-Cote und Z-Cote HP1 von der BASF AG, MZ -300, MZ -500 und MZ-505M von der Fa. Tayca Corporation
- Eisenoxide
Des weiteren ist es vorteilhaft, wenn die mikrofeinen Feststoffteilchen aus der folgenden Gruppe gewählt werden: Bornitride, Stärkederivate (Tapioca Starch, Sodium Corn Starch Octynylsuccinat etc.), Talkum, Latexpartikel.

Es ist erfindungsgemäß vorteilhaft, wenn die feststoffstabilisierten Emulsionen deutlich weniger als 0,5 Gew.-% eines oder mehrerer Emulgatoren enthalten bzw. sogar gänzlich emulgatorfrei sind.

### Stifte

Weiterhin vorteilhaft im Sinne der Erfindung sind Zubereitungen in Form von Stiften. Technisch betrachtet sind die meisten Stiftformulierungen wasserfreie Fettmischungen aus festen oder halbfesten Wachsen und f lüssigen Ö len, wobei hochgereinigte P araffinöle und -wachse die Stiftgrundmasse darstellen.

Übliche Grundstoffe für stiftförmige Zubereitungen sind beispielsweise flüssige Öle (wie z. B. Paraffinöle, Ricinusöl, Isopropylmyristat, C₁₂₋₁₅ Alkylbenzoat), halbfeste Bestandteile (z. B. Vaseline, Lanolin), feste Bestandteile (z. B. Bienenwachs, Ceresin und mikrokristalline Wachse bzw. Ozokerit) und/oder hochschmelzende Wachse (z. B. Carnaubawachs, Candelillawachs). Auch wasserhaltige stiftförmige Zubereitungen sind an sich bekannt, wobei diese auch in Form von W/O-Emulsionen vorliegen können.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutylcarbamate (z. B. die unter den Handelsbezeichnungen Glycacil-L, Glycacil-S von der Fa.

Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder d essen D erivate d as o der d ie A ntioxidantien d arstellen, i st vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder ß-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden k önnen a us d er G ruppe S iliciumdioxid, A luminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 a bgelegt u nd z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten s ind vorteilhaft i m Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 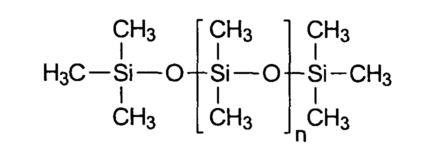 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), weiche n ach INCI a uch als C yclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene A bil-Wax-Typen bei T h. G oldschmidt e rhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäß der vorliegenden Erfindung können ferner vorteilhaft eine oder mehrere Substanzen aus der folgenden Gruppe der S iloxanelastomere enthalten, b eispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise M ethyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich u nd q uellfähig s ind, d ie d urch d ie Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, daß die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als physikalische UV-Filtersubstanzen enthalten, welche in der Wasser- und/oder der Ölphase vorliegen können.

Die Zubereitungen gemäß der vorliegenden Erfindung können ferner vorteilhaft auch in Form von sogenannten ölfreien kosmetischen oder dermatologischen E mulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige U V-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und E ster d er Z imtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - C opolymer welches b eispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), C ers (z. B. Ce₂O₃), M ischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden.

Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen a us A luminiumoxid (Al₂O₃), A luminiumhydroxid Al(OH)₃, b zw. A luminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, i n Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO2) | Octyltrimethylsilan | Degussa |
| Tioveil AQ 10PG | Alumina / Silica | Solaveil / Uniquema |
| Eusolex T-aqua | Wasser/Alumina/Natriummetaphosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 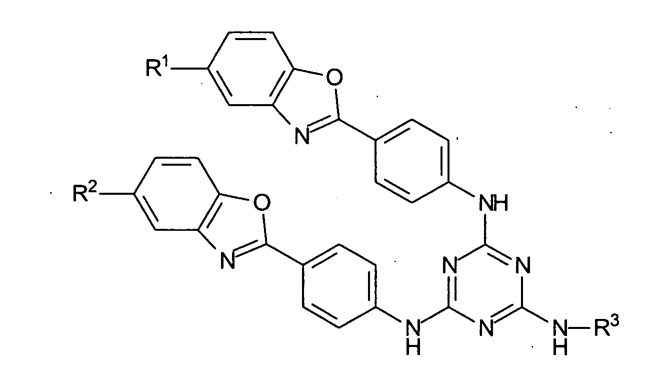 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 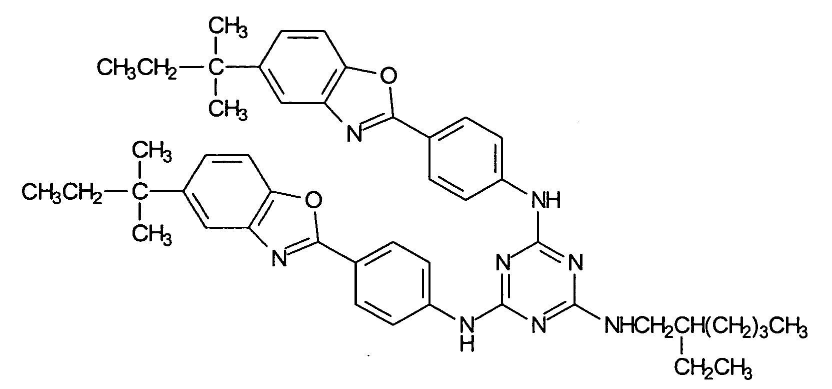 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 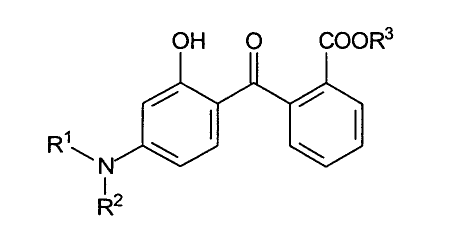 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.
   Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 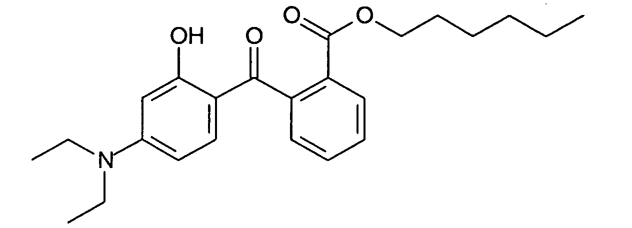 und unter dem Uvinul A Plus bei der Fa. BASF erhältlich ist.
   Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.
   Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4.4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCl: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz eweils einzeln oder in beliebigen Kombinationen miteinander.
Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die Zubereitungen im Sinne der vorliegenden Erfindung können ferner vorteilhaft Substanzen enthalten, die die Wasserfestigkeit der Produkte erhöhen. Insbesondere vorteilhaft sind die im folgenden genannten wasserfestigkeitserhöhenden Substanzen:
A) PEG-45 Dodecylglykolcopolymer (INCI: PEG-45 Dodecyl Glycol Copolymer [y=z=11 und x=45]) und PEG-22 Dodecylglykolcopolymer (INCI: PEG-22 Dodecyl Glycol Copolymer [y=z=4,5 und x=22]) und Methoxy PEG-22 Dodecylglykolcopolymer (INCI: Methoxy PEG-22 Dodecyl Glycol Copolymer [y=7 und x=22 und R = CH₃]) zu verwenden, welche von der Firma AKZO Nobel erhältlich sind.
B) wasserlösliche oder in Wasser dispergierbare Polyoxyethylen-Polyoxypropylen-Blockpolymere (CTFA-Bezeichnung: Polaxamere, CAS-Nr. 9003-11-6) mit folgender Struktur: 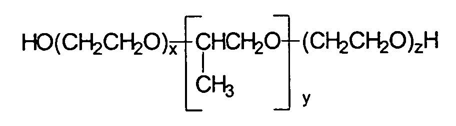 wobei x, y und z ganze Zahlen aus dem Bereich von 2 bis 130, insbesondere von 15 bis 100 darstellen und x und z gleich sind, aber unabhängig von y gewählt werden.
   Unter diesen sind insbesondere Polaxamer 188 [mit x = 75, y = 30 und z = 75], welches unter der Handelsbezeichnung Lutrol F 68 (alt: Pluronic F 68) von der Firma BASF zu beziehen ist, das Polyxamer 185 [mit x = 19, y = 30 und z = 19] (Lubrajel WA von ISP), das Polyxamer 235 [mit x = 27, y = 39 und z = 27] (Pluronic F 85 von BASF) und/oder das Polyxamer 238 [mit x = 97, y = 39 und z = 97] (Pluronic F 88 von BASF) vorteilhaft zu verwenden.
C) acetylierte Stearinsäureester, wie z. B. acetyliertes Glykolstearat. Erfindungsgemäß vorteilhaft kann auch die Kombination von acetyliertem Glykolstearat mit Tristearin (INCI: Acetylated Glycol Stearat and Tristearin) verwendet werden, welche beispielsweise unter der Handelsbezeichnung Unitwix von der Fa. ISP erhältlich ist. Solche Produkte sind unter den Chemical-Abstracts-Registraturnummern 91052-08-3 und 94944-95-3 registriert.
D) Weitere vorteilhafte Substanzen, welche zur Steigerung der Wasserfestigkeit beitragen können, aber in die Ölphase der Zubereitungen gemäß der vorliegenden Erfindung eingearbeitet werden, sind bestimmte Wachskomponenten, wie C₁₈₋₃₆ Fettsäuretriglycerid (z. B: Syncrowax HGLC von der Fa. Crode GmbH mit der INCI: C18-36 Acid Triglyceride) sowie die unter den Handelsbezeichnungen "Performa V 1608" (INCI: C30-38 Olefin/Isopropyl Maleate/MA Copolymer) und "Perfroma V 825" (synethisches Wachs) von New Phase Technologies erhältlichen Substanzen.

Es ist besonders vorteilhaft im Sinne der vorliegenden Erfindung eine oder mehrere der genannten Substanzen zu kombinieren, um die Wasserfestigkeit der Zubereitungen zu verbessern.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1. O/W-Lotion

| | |
|---|---|
| Glyceryl Stearat Citrat | 2.00 |
| Stearylalkohol | 2.00 |
| Octyldodecanol | 10.00 |
| Carbomer | 0.10 |
| Octocrylen | 5.00 |
| Butyl Methoxydibenzoylmethan | 3.00 |
| Titandioxid + Mica | 3.00 |
| Titandioxid + Mica + Eisenoxid | 0.50 |
| Titandioxid + Mica + Zinnoxid | 0.30 |
| Phenoxyethanol | 0.50 |
| Alkohol | 3.00 |
| Propylparaben | 0.07 |
| Methylparaben | 0.25 |
| Glycerin | 3.00 |
| Natriumhydroxid | 0.025 |
| Wasser | Ad 100 |

### 2. O/W-Lotion

| | |
|---|---|
| Glyceryl Stearat Citrat | 2.00 |
| Stearylalkohol | 2.00 |
| Caprylic/Capric Triglycerid | 5.00 |
| Dicaprylylether | 4.00 |
| C10-30 Alkyl Acrylat Crosspolymer | 0.05 |
| Ethylhexyl Methoxycinnamate | 5.00 |
| Butyl Methoxydibenzoylmethan | 3.00 |
| Titandioxid + Mica | 2.00 |
| Titandioxid + Mica + Silica | 1.00 |
| Phenoxyethanol | 0.50 |
| Alkohol | 3 00 |
| Propylparaben | 0.07 |
| Methylparaben | 0.25 |
| Glycerin | 3.00 |
| Natriumhydroxid | 0.025 |
| Wasser | Ad 100 |

### 3. O/W-Lotion

| | |
|---|---|
| Glyceryl Stearat Citrat | 2.00 |
| Stearylalkohol | 2.00 |
| Butylen Glycol Dicaprylat/Dicaprat | 6.00 |
| Dicaprylylether | 4.00 |
| C10-30 Alkyl Acrylat Crosspolymer | 0.05 |
| Ethylhexyl Methoxycinnamate | 5,00 |
| Bisethylhexyloxyphenolmethoxyphenyltriazin | 2.50 |
| Ethylhexyl Triazone | 2.00 |
| Dimethicon | 3.00 |
| Diethylhexyl Butamido Triazone | 1.00 |
| VP Hexadecen Copolymer | 1.00 |
| Titandioxid + Mica | 2.50 |
| Titandioxid + Mica + Eisenoxid | 0.80 |
| Titandioxid + Mica + Aluminiumoxid | 0.60 |
| Vitamin E Acetat | 1.00 |
| Xanthan Gummi | 0.30 |
| Phenoxyethanol | 0.50 |
| Alkohol | 3.00 |
| Propylparaben | 0.07 |
| Methylparaben | 0.25 |
| lodopropynyl Butylcarbamat | 0.01 |
| Glycerin | 3 .00 |
| Natriumhydroxid | 0.025 |
| Parfüm | 0.30 |
| Wasser | Ad 100 |

### 4. Sonnenschaum

| | |
|---|---|
| PEG-40 Stearat | 1.50 |
| Stearinsäure | 2.00 |
| Cetyl Alkohol | 2.00 |
| Ethylhexyl Methoxycinnamat | 4.00 |
| Butyl Methoxydibenzoylmethan | 3.00 |
| Cetyl Ricinoleat | 1.50 |
| Sorbitan Stearat | 1.00 |
| Glycerin | 10.00 |
| Natriumhydroxid | 0.20 |
| Titandioxid + Mica | 0.50 |
| Titandioxid + Mica + Eisenoxid | 4.00 |
| Titandioxid + Mica + Zinnoxid | 2.00 |
| Titandioxid + Mica + Silica | 6.00 |
| Phenoxyethanol | 0.40 |
| Methylparaben | 0.35 |
| Parfüm | 0.40 |
| Wasser | Ad 100 |

### 5. Sprühbare Emulsion

| | |
|---|---|
| Isoceteth-20 | 6.00 |
| Glyceryl lsostearate | 3.50 |
| Paraffinöl | 8.00 |
| Glycerin | 8.00 |
| Dinatriumphenyldibenzimidazol Tetrasulfonat | 1.00 |
| Titandioxid + Mica + Aluminiumoxid | 0.50 |
| Titandioxid + Mica + Silica | 6.00 |
| DMDM Hydantoin | 0.10 |
| Dihydroxyaceton | 6.00 |
| Parfüm | 0.50 |
| Wasser | Ad 100 |

### 6. Pickering-Lotion

| | |
|---|---|
| Mineralöl | 16,00 |
| Octyldodecanol | 5,00 |
| Caprylic/Capric Triglycerid | 6,00 |
| Hydroxyoctacosanyl Hydroxystearat | 1,50 |
| PVP/Hexadecene Copolymer | 1,00 |
| Disteardimonium Hectorite | 1,00 |
| Cyclomethicon | 2,00 |
| Butyl Methoxydibenzoylmethan | 0,50 |
| Ethylhexyltriazon | 1,50 |
| TiO₂ + Alumina + Simethicon + Aqua | 2,00 |
| Zinkoxid | 2,00 |
| Silica Dimethyl Silylat | 0,50 |
| Magnesium Sulfat | 0,70 |
| Glycerin | 3,00 |
| Titandioxid + Mica + Eisenoxid | 3,00 |
| Titandioxid + Mica | 10 ,00 |
| Butylenglycol | 5,00 |
| Trisodium EDTA | 1,00 |
| Propylene Carbonat | 0,33 |
| Methylparaben | 0,21 |
| Propylparaben | 0,07 |
| Phenoxyethanol | 0,50 |
| Wasser | Ad 100 |

### 7. PIT - Emulsionen (Spray)

| | |
|---|---|
| Glycerinmonostearat SE | 0,50 |
| Glyceryl Isostearat | 2,00 |
| Ceteareth-20 | 3,00 |
| Cetyl Alkohol | 1,50 |
| Cetyl Dimethicon Copolyol | 1,00 |
| Titandioxid + Mica + Zinnoxid | 3,00 |
| Titandioxid + Mica + Eisenoxid | 7,00 |
| Terephthaliden Dicampher Sulfonsäure | 1,00 |
| Ethylhexyl Triazon | 1,50 |
| Tocopherol | 1,00 |
| Ethanol | 7,50 |
| Parfüm | 0.20 |
| Wasser | ad. 100 |

### 8. Schutzstift

| | |
|---|---|
| PEG-45/ Dodecyl Glycol Copolymer | 3.00 |
| Polyglyceryl-3 Diisostearat | 2.00 |
| Caprylic/Capric Triglyceride | 5.00 |
| Dicaprylyl Ether | 3.00 |
| Octyldodecanol | 3.00 |
| Cetearylisononanoate | 1.00 |
| Titandioxid + Mica | 4.00 |
| Titandioxid + Mica + Silica | 0.50 |
| C20-40 Alkyl Stearate | 10.00 |
| Hydroxyethylcellulose | 0.50 |
| Butylene Glycol | 4.00 |
| Glycerin | 5.00 |
| Perfume (Parfum) | 1.20 |
| Sodium Corn Starch Octenylsuccinate | 0.60 |
| Water ad. | 100.0000 |

### 9. Hydrodispersionsspray

| | |
|---|---|
| Xanthan Gummmi | 1,00 |
| C10-30 Alkyl Acrylat Crosspolymer | 0.05 |
| Ceteareth-20 | 1.00 |
| Bis-Ethylhexyloxyphenol Methoxyphenyl Triazine | 0,50 |
| Butyl Methoxydibenzoylmethan | 2.50 |
| Bisimidazylat | 0,50 |
| Terephthaliden Dicampher Sulfonsäure | 0,50 |
| Diethylhexyl Butamido Triazone | 3,50 |
| Titandioxid | 4,00 |
| Cocoglycerid | 8,00 |
| Dicaprylyl Carbonat | 10,0 |
| PVP Hexadecen Copolymer | 1,00 |
| Titandioxid + Mica + Silica | 5.00 |
| Titandioxid + Mica | 1.00 |
| Vitamin E -Acetat | 1,50 |
| lodopropyl Butylcarbamat | 0,20 |
| Propylparaben | 0,10 |
| Trisodium EDTA | 0,20 |
| Parfüm | 0,10 |
| Wasser | ad. 100 |

## Patentansprüche

1. Kosmetische oder dermatologische Zubereitung mit einem Gehalt an reflektierenden Festkörpersubstanzen, welche in die drei Raumrichtungen A, B und C die Ausdehnungen a, b und c aufweisen, wobei
• a ≤ 1 µm,
• 10 µm ≤ b ≤ 70 µm und
• 10 µm ≤ c ≤ 70 µm
gewählt werden.

2. Zubereitung nach Anspruch 1, **dadurch gekennzeichnet, dass** die Gesamtmenge an reflektierenden Festkörpersubstanzen aus dem Bereich von 0,01 Gew.-% bis 20 Gew.-% gewählt wird, bezogen auf das Gesamtgewicht der Zubereitungen.

3. Zubereitung nach einem der vorhergehenden Ansprüche, **dadurch gekennzeichnet, dass** sie mindestens eine Selbstbräunungssubstanz enthält.

4. Zubereitung nach einem der vorhergehenden Ansprüche, **dadurch gekennzeichnet, dass** sie mindestens eine Substanz enthält, die UV-Strahlung im UV-A- und/oder UV-B-Bereich absorbiert.

5. Zubereitung nach einem der vorhergehenden Ansprüche, **dadurch gekennzeichnet, dass** sie anorganische Pigmente enthält, die als physikalische UV-Filtersubstanzen dienen.

6. Zubereitung nach einem der vorhergehenden Ansprüche, **dadurch gekennzeichnet, dass** sie wasserfest ist.
